# EUROPEAN PATENT SPECIFICATION

(11) **EP 1 788 926 B1**
(45) Date of publication and mention of the grant of the patent: **02.11.2016**
(21) Application number: 05766759.4
(22) Date of filing: 22.06.2005
(51) Int. Cl.: A61M 25/09, A61B 5/00

(54) **VARIABLE STIFFNESS GUIDEWIRE**
FÜHRUNGSDRAHT MIT VARIABLER STEIFHEIT
FIL-GUIDE DE RAIDEUR VARIABLE

(30) Priority: 22.06.2004 US 582033 P
(43) Date of publication of application: 30.05.2007
(73) Proprietor: LAKE REGION MANUFACTURING, INC., Chaska, Minnesota 55318 (US)
(72) Inventor: MINAR, Chris, New Prague, MN 56071-9106 (US); DOUGLAS, Jeanne, Shakopee, MN 55379-3920 (US); SENN, Andrew, Chaska, Minnesota 55318 (US)
(74) Representative: Parry, Simon James
(86) International application number: PCT/US2005/021985
(87) International publication number: WO 2006/002199

(56) References cited:
- EP-A- 0 515 201
- EP-A- 0 784 991
- EP-A- 1 426 071
- US-A1- 2002 062 092
- US-A1- 2003 069 521
- US-A1- 2004 087 876
- US-A1- 2004 087 876
- US-A1- 2004 193 073

## Description

In general, guidewires for medical procedures such as angioplasty, are made with stainless steel wire (SS) which, when ground in a series of tapers and traights, generally decreases from a proximal core body diameter of 0,36 mm (.014 inches) down to a distal diameter of approximately 0,05 mm (.002 inches). A portion of this ground section of core, possibly including a ribbon wire, will be covered with one or more coils.

Some disclosures describe guidewire with nitinol distal portions that yield at a desired set shape (US 5,238,004) and consist of a SS proximal section attached to a linear elastic distal section. This layout of the '004 patent has a lower kink resistance than the proposed invention.

Other disclosures (e. g., US 6,592,570) involve a SS proximal section and a distal portion that is entirely superelastic. This superelastic distal tip restricts shapeability and can cause the placement of the guidewire into a vessel to be difficult. The proposed invention is kink resistant, yet it has a shapeable tip that will assist the user in negotiation of vessels.

EP-A-1426071 discloses a so-called "foreshortened core wire" guidewire device, whose core wire terminates short of the guidewire tip, such that no core wire distal portion stiffness variation is possible.

A stainless steel body proximal guidewire gives exceptional column strength to push, support catheters, and torque the distal end of the guidewire through the vessel. A nitinol ground middle to distal section is flexible enough to avoid damaging the vessel during advancement of the guidewire. The shortcoming to a stainless steel distal section is that it can bend and kink during advancement, making it difficult to advance into the chosen vessel. Current guidewires made of a SS proximal portion and superelastic nitinol distal portion have a transition in a step or discrete fashion, making for potentially difficult navigation. Other guidewires made of a SS proximal section and a linear elastic nitinol distal section transition more smoothly, but are less kink resistant
at the distal tip. For these reasons a kink resistant guidewire that decreases gradually in stiffness from the proximal to the distal end *i.e*., a variable or gradually decreasing stiffness guidewire, is ideal.

### SUMMARY OF THE OF INVENTION

By attaching a nitinol distal ground section to the stainless steel proximal core wire or element of a guidewire the propensity of the distal flexible section to bend and kink is reduced. This invention includes a highly torqueable, pushable guidewire with a relatively stiff proximal (preferably in one embodiment), stainless steel (SS) section and kink resistant nitinol medial to distal section attached to a shapeable SS, in one embodiment, (such as a SS ribbon) distal tip. The very distal SS tip will have the ability to be shaped by the end user in order to facilitate entry into the chosen vessels.

It is the purpose of this invention to provide an improved, kink resistant guidewire. This is best achieved by gradually decreasing the stiffness from the proximal to the distal end by using, for example, a combination of SS and variably heat-treated nitinol.

The novelty of this invention involves the combination of different metals and elementally similar metals in different phases to attain ideal guidewire characteristics.

Thus, in one embodiment, the present invention is a variable stiffness guidewire comprising:
a core element or core wire having proximal and distal portions, the distal portion having proximal and distal segments, the distal segment of the core element comprising nitinol, the nitinol being at least partially linear elastic in the proximal segment of the distal portion, and super elastic in the distal segment of the distal portion.

In a further embodiment, the present invention is a variable stiffness guidewire comprising a core element or wire having a proximal portion and a distal portion, the distal portion having distal and proximal segments and the proximal portion having distal and proximal segments:
the core element proximal portion comprising stainless steel;
the core element distal portion comprising;
a superelastic distal portion and a linear elastic proximal portion, the core element having attached to its extreme distal end;
an atraumatic tip.

In yet another embodiment, the present invention is a variable stiffness guidewire comprising a core element having proximal and distal portions comprising different materials:
the proximal portion including a reduced-diameter distal section;
the distal portion including a reduced-diameter proximal section;
a hollow coupler connecting the reduced-diameter section of the proximal portion to the reduced-diameter section of the distal portion, wherein the proximal portion comprises a non-super elastic alloy; and the distal portion comprises nitinol in a linear elastic state on its proximal portion and a nitinol in super elastic reduced-diameter distal portion.

Stiffness is variable or is gradually decreased or decreasing by assembling a SS proximal section to a nitinol distal section, which is attached to an independent or attached SS tip. More specifically, the nitinol distal portion comprises a linear elastic nitinol proximal segment and a super elastic nitinol distal segment. The nitinol distal segment is attached to, *e.g*., an SS proximal portion or segment. This graduation from linear elastic nitinol to superelastic nitinol creates an advantageous, gradual, and controlled or controllable reduction or decrease in guidewire stiffness (in accordance with this invention) when moving distally from the proximal segment to the distal segment to create a kink resistant guidewire. The combination of this two-phase, distal nitinol section and the stiff proximal 304 stainless steel section along with the optional shapeable SS distal portion (*i.e*., a shaping ribbon) creates the ideal torqueable, pushable, and steerable guidewire having varying or distally-decreasing stiffness.

### BRIEF DESCRIPTION OF THE DRAWINGS

FIG. 1 where shown, in section, a finger-formable version (not comprised within the scope of the claims) in which the superelastic distal segment of the core wire or core element extends distally from before the first taper (shown by shading) all the way to the end of the core wire, which terminates short of the extreme distal tip;
FIG. 2 is an embodiment (not comprised within the scope of the claims) in which the superelastic distal portion begins adjacent to the first core wire taper (shown by shading) and extends to the end of the core wire, which also terminates short of the guidewire distal tip;
FIG. 3 is an embodiment (not comprised within the scope of the claims) in which the superelastic core wire segment begins distal to the first core wire taper (shown by shading) and extends to the end of the core wire which also terminates short of the guidewire extreme distal tip;
FIG. 4, FIG. 5 and FIG. 6 are non-tip formable embodiments of the present invention in which the zones of superelastic core wire begin at the same points as shown in FIGs. 1, 2 and 3, respectively and the core wire or core element extends all the way to the guidewire tip.
FIG. 7 shows in section an embodiment to the present invention in which the superelastic distal segment extends all the way to the extreme distal tip of the guidewire and in which the guidewire is also tip formable.
FIG. 8 is a stress/strain curve (to break) of the materials indicated thereon.
FIG. 9 is displacement vs. load.

### DETAILED DESCRIPTION OF THE INVENTION

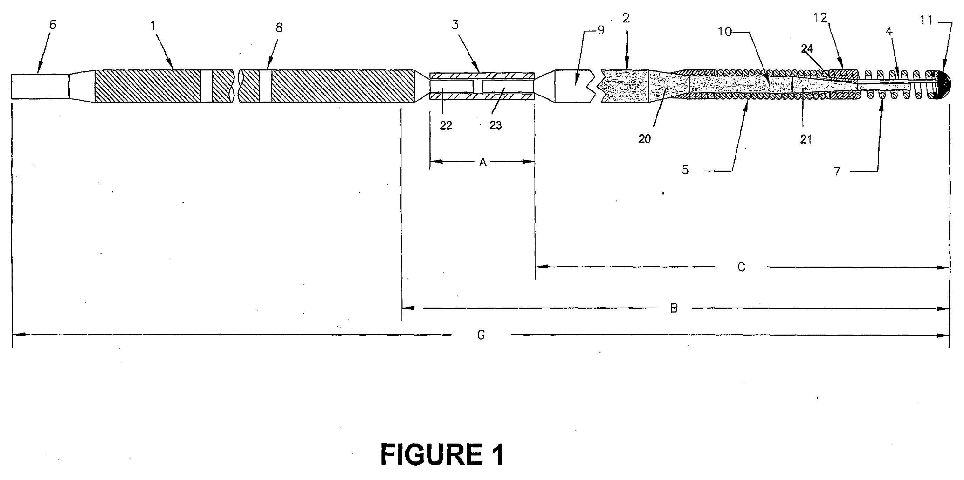

This invention is, in one embodiment, a Dual Phase Nitinol PTCA Guidewire (see attached FIGs. 1-7) with a stainless steel proximal shaft 1 and a nitinol medial to distal section 2 (generally, the length indicated by "C") attached by a nitinol hypo tube 3. This nitinol middle section is then attached to a SS distal tip 11. Distal tip 11 is attached to the nitinol section 2 by means of a spring coil 5, 7 and a ribbon wire or safety ribbon 4. Spring coil 5, 7 comprises a radiopaque segment 7 and a non-radiopaque segment 5 which overlap or are interwound and are soldered together at 12. The embodiment shown has two tapered or ground section 20, 21. Other combinations of tapered, ground, or flattened guidewire sections are within the contemplation of this invention.

The invention encompasses a range of constructions from floppy to extra support grind configurations. The invention preferably is built in 190 cm length and 300 cm length configurations with a preferred maximum diameter of .014". The proximal core segment 1 is preferably PTFE coated and the distal segment 2 preferably is coated with a separate lubricious coating e.g., silicone, or other hydrophilic coating. The hydrophilic coating commercially available from Surmodics Corp. is preferred. The invention contemplates an optional extension system to be used with the 190 cm wire version having a proximal connector structure 6. As was noted a platinum distal marker coil 7 is threaded into a coil spring 5 and optional proximal depth marks on the optional PTFE-coated proximal section 8 are placed using e.g., an emulsion ink.

The nitinol middle section "C" will consist of a proximal linear elastic segment 9, which will graduate into a superelastic (heat-treated) segment 10 indicated by shading in all the FIGURES. The superelastic section 10 is created by placing the desired length of core wire segment in an oven at about 1000°F for 20 minutes. There will be a slight transition created between the linear elastic and superelastic segments that will aid in a gradual transition of lowered stiffness contributing to the variable stiffness feature of this invention. One skilled in this art will appreciate that either or both of the time of treatment or temperature of treatment of the core wire may be adjusted to obtain the requisite superelasticity. This transition length can be easily optimized to improve the overall properties of the guidewire. Other linear elastic sections may be heat-treated at lower temperatures to create more elasticity/less stiffness and a more gradual transition. (see attached test data for stiffness variations)

It has also been determined that the nitinol wire section 2 included in this invention has the ability to be straightened mechanically or by providing tension during the heat-treatment. These operations also create variable elasticity and could be used in the gradual reduction of stiffness along the guidewire.

A process has been developed that involves the heat treatment of linear elastic NiTi. The linear elastic core is placed in an oven at about 950 F for 25 minutes with no longitudinal tension which causes the extreme distal portion of the guidewire to become superelastic. The oven is constructed in a way so that there is a void or hole in the wall of the oven where a wire can be placed. The NiTi core is inserted in to the void so that the desired length of super elastic NiTi is fully inside the oven. The heat dissipation leaving the void of the oven produces a smooth stiffness and elasticity transition from the linear to superelastic region. Stress vs. strain results, have been measured and a gradual drop in stiffness from the linear elastic region to the superelastic region is clearly apparent using the above-described process. In this manner, the overall stiffness of the guidewire may be controlled by controlling the length of the core wire superelastic segment.

Thus, there is shown at FIGURES 1-7, in section, several embodiments of the present invention. It is to be understood that in each of these embodiments a distal portion of a guidewire core wire 2 comprises a more distal superelastic segment 10 and a more proximal linear elastic segment 9. In a preferred embodiment, the distal core wire or element comprises a nickel titanium alloy having a composition in the range of from about 54 atomic % nickel to 46 atomic % titanium to about 57 atomic % nickel to about 43 atomic % titanium.

In FIG. 1 there is shown an embodiment of the invention in which there is a hydrophobically-coated, e.g., PTFE, stainless steel proximal guidewire/core wire segment and a distal linear elastic 9/super elastic 10, (shown by shading) core wire segment 2, the core wire terminating short of the extreme distal tip of the guidewire. The hydrophobic coating on the proximal segment of the guidewire preferably comprises PTFE. Various other hydrophobic coatings will readily come to mind to one skilled in this art in view of this disclosure. As is shown, the extreme proximal portion of the proximal guidewire segment may be coated (shorter lengths) or lack coating so as to permit the guidewire to be more easily handled.

The proximal portion (approximately the "G" dimension minus the "B" dimension) and distal segment 2 are shown to be coupled by a hypotube connector 3, each such segments having reduced diameter portions 22, 23 which are inserted into opposite ends of the hypotube connector 3 and are bonded thereto e.g., by the use of solder or glue. The hypotube connector 3 may comprise stainless steel, linear elastic or superelastic alloys depending upon design preference. Other equivalent means of creating a coupler between a stainless steel proximal segment and a linear elastic/super elastic distal segment will occur to one skilled in this art in view of the present disclosure c.f., U.S. Patent 5,341,818 Abrams et al..

There are other guidewire optional features shown in FIG. 1. For example, a spring coil is shown to be attached to the first core wire taper on its proximal end and to the guidewire tip 11 on the distal end. The spring coil extends distally to a point where the stainless steel coil 5 ("SS coil") is inter-wound with a platinum core wire 7 and soldered or glued thereto 12. As is shown the platinum coil wire is inter-wound with the stainless steel coil a length sufficient to provide an aggressive yet flexible (so as not to create a flat spot) connection. Also optionally included in this embodiment of the invention is a generally flat stainless steel ribbon wire 4 soldered (or otherwise attached) to the core wire which extends distally to the atraumatic, extreme guidewire tip. The ribbon wire imparts finger formability to the distal segment of the guidewire.

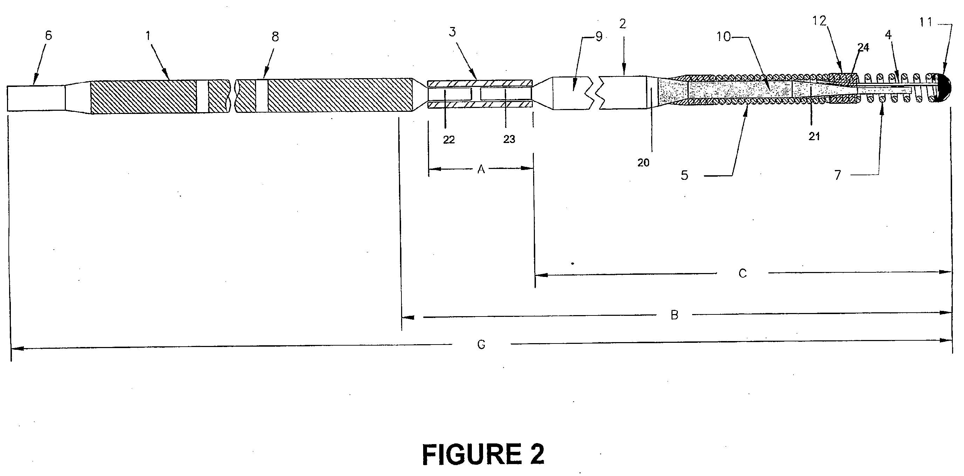

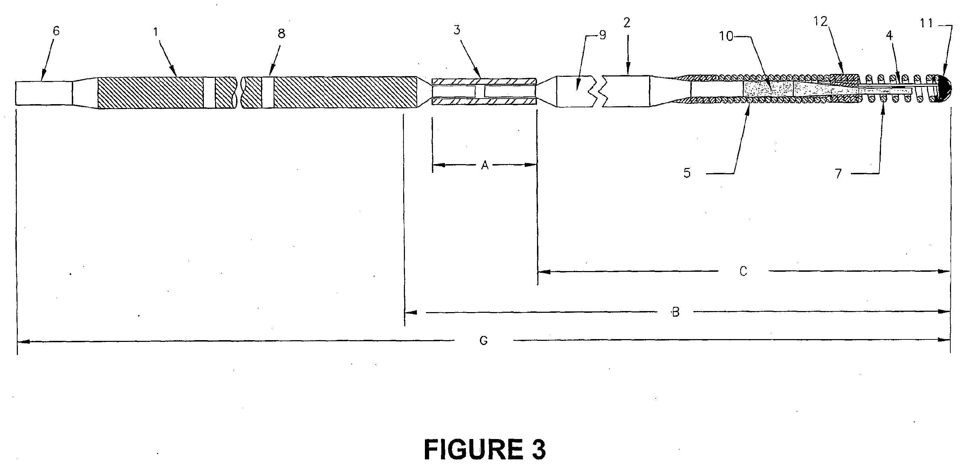

FIGs. 2 and 3 depict variations of the invention shown in FIG. 1, the primary difference being that the shaded superelastic segment 10 begins at a zone or region that is distally-disposed relative to the comparable superelastic segment shown in FIG. 1. In FIG. 2 the superelastic segment 10 begins adjacent the first taper 20 of the guidewire distal segment. In FIG. 3 the superelastic segment 10 (shaded), is distally located from the comparable structures shown on FIGs. 1 and 2. The shorter superelastic zones or portions of the guidewires shown in FIGs. 2 and 3 mean that the distal portion of the guidewire is increasingly less "floppy" or conversely, stiffer. In each of the guidewires depicted in FIGs. 1, 2 and 3 a finger-formable ribbon wire 4 is used to impart finger formability to the distal segment of the guidewire. In this manner the medical professional using the guidewire may impart a desired bend or deviation from linearity, which will be retained by the guidewire, depending upon the user's personal preference.

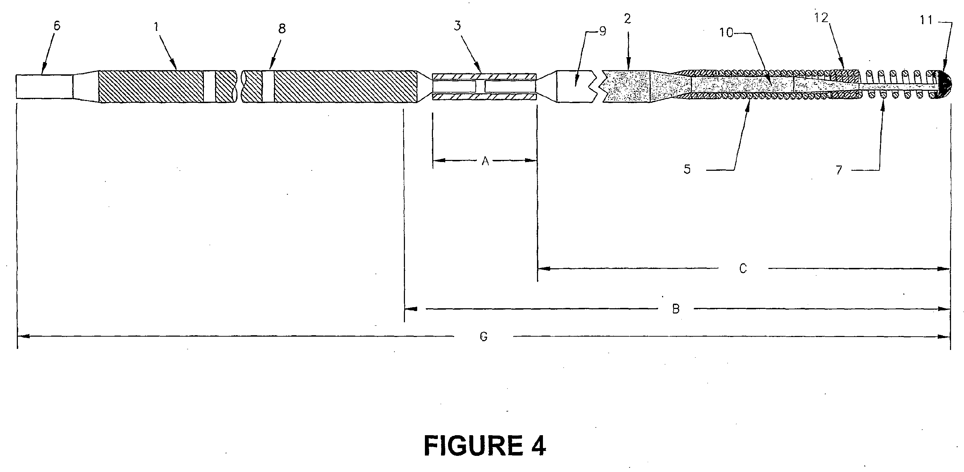

FIG. 4 depicts the guidewire of the present invention in which the distal portion of the guidewire does not have the optional flat stainless steel ribbon wire or safety wire extending between the guidewire core and the atraumatic guidewire extreme distal tip. Also, the superelastic core wire or core element extends all the way to the atraumatic tip of the guidewire and is surrounded by a stainless steel/platinum spring coil 5, 7. The absence of the ribbon wire/safety wire means that the distal portion of the guidewire shown in FIG. 4 will not be easily finger formable. Put otherwise, the superelastic characteristics of the distal portion of the core wire will prevent it from easily retaining a bend or lateral displacement imparted thereto.

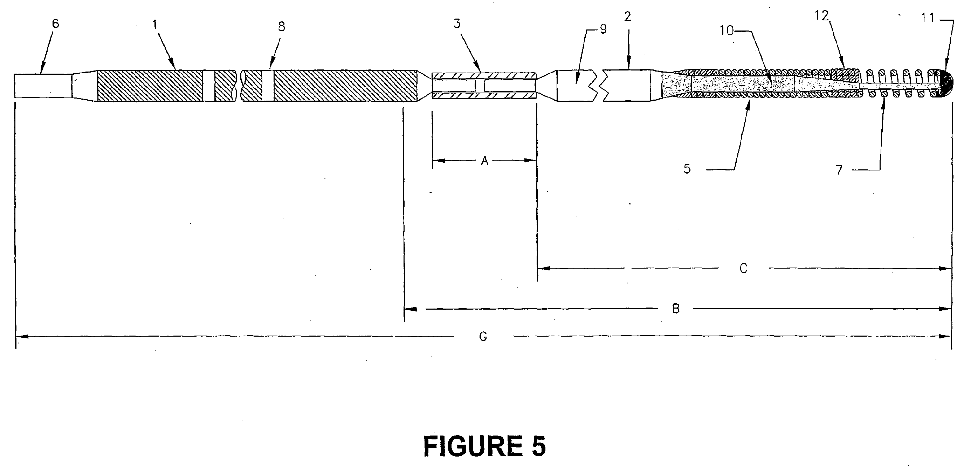

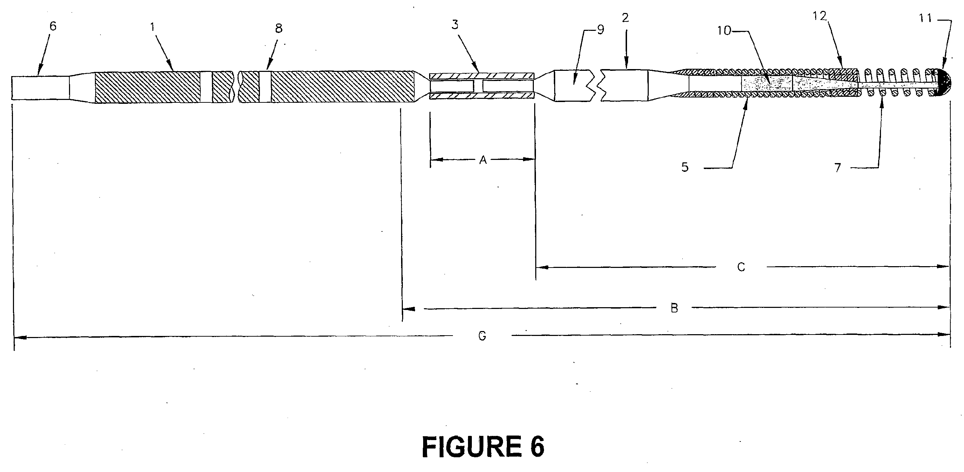

FIGs. 5 and 6 depict the guidewire shown in FIG. 4 with distally-disposed, shorter superelastic core wire segments 10. All other features of the guidewires of FIGs. 5 and 6 are the same as that of FIG. 4.

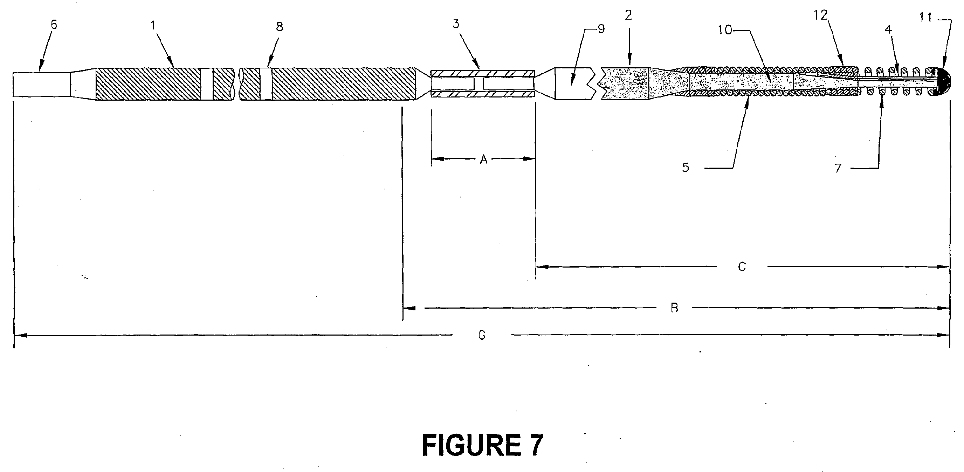

FIG. 7 shows it a further embodiment to the present invention in which the superelastic core wire extends all the way to the atraumatic distal tip and a ribbon wire or safety wire 4 also is employed. The guidewire shown in FIG. 7 is otherwise the same as the guidewires shown in FIGs. 1-6.

It is to be understood that the controllable or variable stiffness of a guidewire of the present invention may also be varied or adjusted by employing one or more distal tapers. The guidewires in FIGs. 1-7 all are shown to employ two such tapers 20,21. Clearly, the number of tapers employed, their location and their length is a matter of design preference depending upon the extent of stiffness (or flexibility) to be imparted to the guidewire.

It is also to be understood that the dimensions shown for the guidewires in FIGs. 1-7 are not to be employed to limit the scope of the present invention. Without being limited thereto, dimensions shown by the use of letters in FIGs. 1-7 generally fall in the following range(s):

**Drawing Letter**

| **Designation** | **Structure** | **Range** |
|---|---|---|
| A. | Length of hypotube | 2-10cm |
| B. | Length of hydrophilic coat | 2-100 cm |
| C. | Length of guidewire distal segment | 10-60cm |
| G. | Overall guidewire length | 70-320cm |

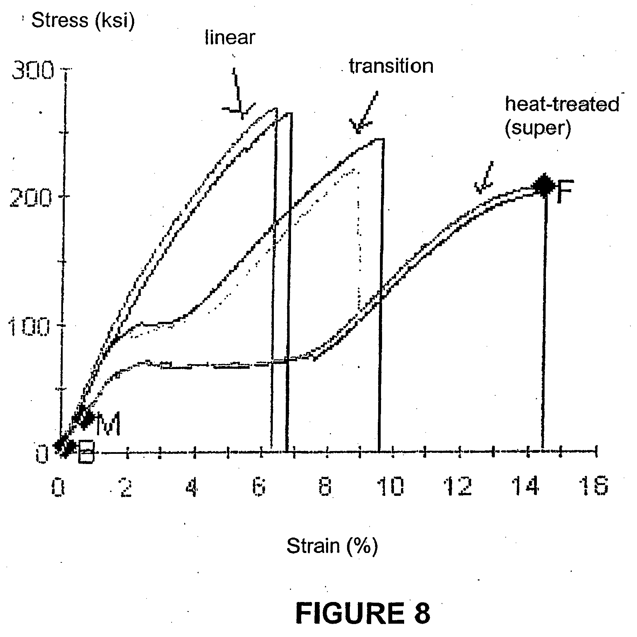

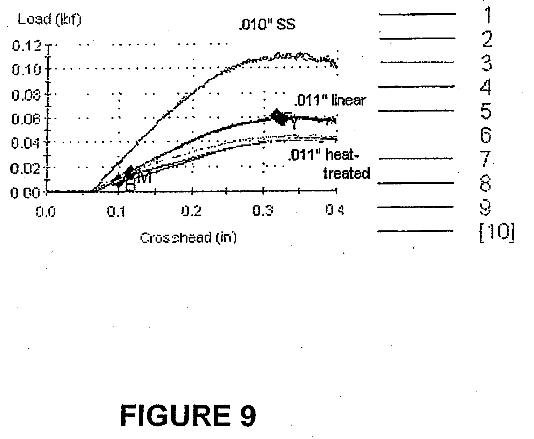

FIGs. 8 and 9 show the relationship between, non-super elastic, super elastic and linear elastic core wire or core element materials as a function of stress vs. strain (to break) FIG. 8, and load vs. displacement (cross head inches). Those FIGURES show the definite performance differences between the alloys and alloy states used in the present invention.

The above invention has been described with particular reference to the use of a nickel/titanium alloy to create the guidewire of variable stiffness herein described. The present invention should not be understood to be limited only to the use of nickel/titanium alloys. In fact, any alloy exhibiting the superelastic/linear elastic characteristics of nickel/titanium alloys employed herein is clearly contemplated. Thus, the present invention is not, and should not, be construed to be limited to the preferred nickel/titanium alloys extensively discussed herein.

## Claims

1. A variable stiffness guidewire comprising a core wire having a proximal portion (1) and a distal portion (2), the distal portion (2) having distal and proximal segments (10, 9) and the proximal portion (1) having distal and proximal segments;
the core wire proximal portion (1) comprising stainless steel; and the core wire having an atraumatic tip (11) attached to its extreme end, the guidewire being **characterised by**:
the core wire distal portion comprising;
a superelastic distal segment (10) and a linear elastic proximal segment (9);
wherein the core wire extends from the core wire proximal portion (1) to the atraumatic tip (11) and is attached to and contacts the atraumatic tip (11) at the core wire's extreme distal end.

2. A guidewire according to claim 1 which further includes a coil spring (5, 7) attached to the distal portion (2) of the core wire and to the tip (11).

3. A guidewire according to claim 2 which further includes a non-super elastic shaping ribbon (4) disposed within the coil spring (5, 7) and attached to the distal portion (2) of the core wire and the tip (11).

4. A guidewire according to claim 3 wherein the shaping ribbon (4) comprises stainless steel.

## Patentansprüche

1. Führungsdraht mit variabler Steifigkeit, welcher einen Kerndraht umfasst, welcher einen proximalen Abschnitt (1) und einen distalen Abschnitt (2) aufweist, wobei der distale Abschnitt (2) ein distales und ein proximales Segment (10,9) aufweist und der proximale Abschnitt (1) ein distales und ein proximales Segment aufweist;
wobei der proximale Abschnitt (1) des Kerndrahts Edelstahl umfasst und der Kerndraht eine atraumatische Spitze (11) aufweist, welche an dessen äußerstem Ende befestigt ist, wobei der Führungsdraht **dadurch gekennzeichnet ist, dass**:
der distale Abschnitt des Kerndrahts Folgendes umfasst:
ein superelastisches distales Segment (10) und ein lineares, elastisches proximales Segment (9);
worin sich der Kerndraht vom proximalen Abschnitt (1) des Kerndrahts zur atraumatischen Spitze (11) erstreckt und an dem äußersten distalen Ende des Kerndrahts an der atraumatischen Spitze (11) befestigt ist und diese dort kontaktiert.

2. Führungsdraht nach Anspruch 1, welcher ferner eine Spiralfeder (5, 7) umfasst, die am distalen Abschnitt (2) des Führungsdrahtes und an der Spitze (11) befestigt ist.

3. Führungsdraht nach Anspruch 2, welcher ferner ein nicht superelastisches Formband (4) umfasst, welches innerhalb der Spiralfeder (5, 7) angeordnet und am distalen Abschnitt (2) des Kerndrahts und an der Spitze (11) befestigt ist.

4. Führungsdraht nach Anspruch 3, worin das Formband (4) Edelstahl umfasst.

## Revendications

1. Fil-guide à rigidité variable comprenant une armature ayant une partie proximale (1) et une partie distale (2), la partie distale (2) ayant des segments distal et proximal (10, 9) et la partie proximale (1) ayant des segments distal et proximal ;
la partie proximale d'armature (1) comprenant de l'acier inoxydable ; l'armature ayant une pointe atraumatique (11) fixée à son extrémité extrême, le fil-guide étant **caractérisé par** :
la partie distale d'armature qui comprend :
un segment distal super élastique (10) et un segment proximal élastique linéaire (9) ;
dans lequel l'armature s'étend à partir de la partie proximale d'armature (1) jusqu'à la pointe atraumatique (11) et est fixée à et est en contact avec la pointe atraumatique (11) au niveau de l'extrémité distale extrême de l'armature.

2. Fil-guide selon la revendication 1, comprenant en outre un ressort hélicoïdal (5, 7) fixé à la partie distale (2) de l'armature et à la pointe (11).

3. Fil-guide selon la revendication 2, qui comprend en outre un ruban de formage non super élastique (4) disposé à l'intérieur du ressort hélicoïdal (5, 7) et fixé à la partie distale (2) de l'armature et de la pointe (11).

4. Fil-guide selon la revendication 3, dans lequel le ruban de formage (4) comprend de l'acier inoxydable.
